# EUROPEAN PATENT APPLICATION

(11) **EP 4 691 628 A1**
(43) Date of publication of application: **11.02.2026**
(21) Application number: 24780731.6
(22) Date of filing: 28.03.2024
(51) Int. Cl.: B01J 23/58, B01J 37/02, B01J 37/16, C07B 61/00, C07C 209/72, C07C 211/18

(54) **CATALYST, METHOD FOR PRODUCING CATALYST, AND METHOD FOR PRODUCING BIS (AMINOMETHYL) CYCLOHEXANE**

(30) Priority: 31.03.2023 JP 2023058039
(71) Applicant: MITSUBISHI GAS CHEMICAL COMPANY, INC., Chiyoda-ku Tokyo 100-8324 (JP)
(72) Inventor: TAKENAKA, Makoto, Niigata-shi, Niigata 950-3112 (JP); IIDA, Akifumi, Niigata-shi, Niigata 950-3112 (JP)
(74) Representative: Müller-Boré & Partner Patentanwälte PartG mbB
(86) International application number: PCT/JP2024/012867
(87) International publication number: WO 2024/204633

(57) **Abstract**

Provided is a catalyst for producing bis(aminomethyl)cyclohexane, the catalyst having hydrogenation ability, wherein an amount at a desorption peak measured for the catalyst by the following ammonia temperature-programmed desorption method is 0.020 mmol/g-cat or less: (ammonia temperature-programmed desorption method) He gas is brought into contact with 150 mg of the catalyst at 500°C at a gas flow rate of 20 sccm for 30 minutes; subsequently, a mixed gas of 5% by volume of NH₃ and balance He is brought into contact with the catalyst at 50°C at a gas flow rate of 20 sccm for 10 minutes; and then, while He gas is brought into contact with the catalyst at a gas flow rate of 20 sccm, the temperature is increased at 10°C/min, followed by the detection of the desorption peak with a thermal conductivity detector.

## Description

### Technical Field

The present invention relates to a catalyst, a method for producing the catalyst, and a method for producing bis(aminomethyl)cyclohexane.

### Background Art

Bis(aminomethyl)cyclohexane (hereinafter, also referred to as "BAC") is an industrially important compound as a starting material for polyamide, bis(isocyanatomethyl)cyclohexane, and the like. BAC can be obtained by, for example, the catalytic hydrogenation of xylylenediamine. As for such catalytic hydrogenation, for example, Patent Literature 1 has proposed that a catalyst prepared by adding an alkali metal modifier serving as an auxiliary agent to a catalytic component ruthenium/alumina is used in the catalytic hydrogenation.

### Citation List

### Patent Literature

Patent Literature 1: Chinese Patent Application Publication No. 112473663

### Summary of Invention

### Technical Problem

The catalyst described in Patent Literature 1 reportedly improves selectivity, however, has a room for further improvement because of recent tendency for demanding higher catalyst performance.

The present invention has been made in light of the problem described above. An object of the present invention is to provide a catalyst that exerts excellent BAC selectivity, for example.

### Solution to Problem

The present inventors have conducted diligent studies and consequently found that the problem can be solved by a catalyst having predetermined physical properties, leading to the completion of the present invention.

Specifically, the present invention encompasses the following aspects.
[1] A catalyst for producing bis(aminomethyl)cyclohexane, the catalyst having hydrogenation ability, wherein
   an amount at a desorption peak measured for the catalyst by the following ammonia temperature-programmed desorption method is 0.020 mmol/g-cat or less:
   (Ammonia temperature-programmed desorption method)
   He gas is brought into contact with 150 mg of the catalyst at 500°C at a gas flow rate of 20 sccm for 30 minutes; subsequently, a mixed gas of 5% by volume of NH₃ and balance He is brought into contact with the catalyst at 50°C at a gas flow rate of 20 sccm for 10 minutes; and then, while He gas is brought into contact with the catalyst at a gas flow rate of 20 sccm, the temperature is increased at 10°C/min, followed by the detection of the desorption peak with a thermal conductivity detector.
[2] The catalyst according to [1], wherein
   the catalyst comprises a catalytic component and a support that supports the catalytic component, and
   the catalytic component comprises Ru.
[3] The catalyst according to [1] or [2], wherein
   a Ru content in the catalyst is 0.1 to 10.0% by mass based on 100% by mass of the catalyst, and
   a Na content in the catalyst is 0.1 to 10.0% by mass based on 100% by mass of the catalyst.
[4] The catalyst according to [2] or [3], wherein the support comprises alumina.
[5] A method for producing the catalyst according to any of [1] to [4], comprising:
   a step (a) of reacting a catalytic component with a support to obtain a first reaction product; and
   a step (b) of adding an alkali metal to the first reaction product to obtain a second reaction product.
[6] The method for producing the catalyst according to [5], further comprising
   a step (c) of reducing the second reaction product to obtain a third reaction product, wherein
   an amount at a desorption peak measured by the ammonia temperature-programmed desorption method for the third reaction product is 0.020 mmol/g-cat or less.
[7] The method for producing the catalyst according to [6], wherein the step (c) is performed at a temperature of 160 to 360°C.
[8] The method for producing the catalyst according to any of [5] to [7], wherein the step (a) comprises adsorbing a Ru starting material solution onto the support, then drying the support, and bringing the support into contact with a basic aqueous solution.
[9] The method for producing the catalyst according to any of [5] to [7], wherein the alkali metal comprises at least one selected from the group consisting of Na, K, and Li.
[10] A method for producing bis(aminomethyl)cyclohexane, comprising a hydrogenation step of continuously bringing xylylenediamine and hydrogen into contact with each other in the presence of a catalyst having hydrogenation ability to obtain bis(aminomethyl)cyclohexane, wherein
   an amount at a desorption peak measured for the catalyst by the following ammonia temperature-programmed desorption method is 0.020 mmol/g-cat or less:
   (Ammonia temperature-programmed desorption method)
   He gas is brought into contact with 150 mg of the catalyst at 500°C at a gas flow rate of 20 sccm for 30 minutes; subsequently, a mixed gas of 5% by volume of NH₃ and balance He is brought into contact with the catalyst at 50°C at a gas flow rate of 20 sccm for 10 minutes; and then, while He gas is brought into contact with the catalyst at a gas flow rate of 20 sccm, the temperature is increased at 10°C/min, followed by the detection of the desorption peak with a thermal conductivity detector.
[11] The method for producing bis(aminomethyl)cyclohexane according to [10], wherein
   the catalyst comprises a catalytic component and a support that supports the catalytic component, and
   the catalytic component comprises Ru.
[12] The method for producing bis(aminomethyl)cyclohexane according to [11], wherein
   the catalyst is obtained by a production method (A), and
   the production method (A) comprises:
      a step (a) of reacting the catalytic component with the support to obtain a first reaction product; and
      a step (b) of adding an alkali metal to the first reaction product to obtain a second reaction product.
[13] The method for producing bis(aminomethyl)cyclohexane according to [12], wherein
   the production method (A) further comprises a step (c) of reducing the second reaction product to obtain a third reaction product, wherein
   an amount at a desorption peak measured by the ammonia temperature-programmed desorption method for the third reaction product is 0.020 mmol/g-cat or less.
[14] The method for producing bis(aminomethyl)cyclohexane according to [13], wherein the step (c) is performed at a temperature of 160 to 360°C.
[15] The method for producing bis(aminomethyl)cyclohexane according to any of [12] to [14], wherein the step (a) comprises adsorbing a Ru starting material solution onto the support, then drying the support, and bringing the support into contact with a basic aqueous solution.
[16] The method for producing bis(aminomethyl)cyclohexane according to any of [12] to [15], wherein the alkali metal comprises at least one selected from the group consisting of Na, K, and Li.
[17] The method for producing bis(aminomethyl)cyclohexane according to any of [10] to [16], wherein
   a Ru content in the catalyst is 0.1 to 10.0% by mass based on 100% by mass of the catalyst, and
   a Na content in the catalyst is 0.1 to 10.0% by mass based on 100% by mass of the catalyst.
[18] The method for producing bis(aminomethyl)cyclohexane according to any of [11] to [17], wherein the support comprises alumina.

### Advantageous Effect of Invention

The present invention can provide a catalyst that exerts excellent BAC selectivity, for example.

### Brief Description of Drawings

[Figure 1] Figure 1 is a graph showing TPD measurement results of a catalyst obtained in Example 1.
[Figure 2] Figure 2 is a graph showing results of the multipoint baseline correction of the graph of Figure 1.
[Figure 3] Figure 3 is a graph showing TPD measurement results of a catalyst obtained in Comparative Example 1.
[Figure 4] Figure 4 is a graph showing results of the multipoint baseline correction of the graph of Figure 3.
[Figure 5] Figure 5 is a graph showing TPD measurement results of a catalyst obtained in Comparative Example 2.
[Figure 6] Figure 6 is a graph showing results of the multipoint baseline correction of the graph of Figure 5.
[Figure 7] Figure 7 is a graph showing TPD measurement results of a catalyst obtained in Comparative Example 3.
[Figure 8] Figure 8 is a graph showing results of the multipoint baseline correction of the graph of Figure 7.

### Description of Embodiments

Hereinafter, the embodiment for carrying out the present invention (hereinafter, referred to as the "present embodiment") will be described in detail. The present embodiment given below is an illustration for describing the present invention and does not intend to limit the present invention to the contents given below. The present invention can be carried out through changes or modifications made without departing from the spirit of the present invention.

### <Catalyst>

The catalyst of the present embodiment is a catalyst for producing bis(aminomethyl)cyclohexane, the catalyst having hydrogenation ability, wherein an amount at a desorption peak measured for the catalyst by the following ammonia temperature-programmed desorption method (hereinafter, also referred to as "TPD") (hereinafter, this measurement is also referred to as "TPD measurement") is 0.020 mmol/g-cat or less:
(Ammonia temperature-programmed desorption method)
He gas is brought into contact with 150 mg of the catalyst at 500°C at a gas flow rate of 20 sccm for 30 minutes; subsequently, a mixed gas of 5% by volume of NH₃ and balance He is brought into contact with the catalyst at 50°C at a gas flow rate of 20 sccm for 10 minutes; and then, while He gas is brought into contact with the catalyst at a gas flow rate of 20 sccm, the temperature is increased at 10°C/min, followed by the detection of the desorption peak with a thermal conductivity detector.

The catalyst of the present embodiment thus configured exerts excellent BAC selectivity.

### (TPD measurement)

The catalyst of the present embodiment exerts excellent BAC selectivity because the amount at a desorption peak measured by the TPD measurement is 0.020 mmol/g-cat or less. The reason for this is not necessarily clear and is presumed as follows, though not intending to limit the reason herein.

The present inventors have conducted studies on conventional hydrogenation catalysts for BAC production and consequently found that hydrogenation reaction tends to cause impurities having low boiling points as by-products and thereby has limitations on improvement in BAC selectivity. As a result of further conducting studies on this cause, the present inventors have assumed that, in particular, acidic sites on surface of catalyst largely influence hydrogenation reaction for BAC production and are strongly related to the generation of the impurities, and thus assumed that further improvement in BAC selectivity is expected by decrease therein. On this assumption, the present inventors have pursued studies and consequently found that, in particular, BAC selectivity is improved when the amount at a desorption peak obtained by the present measurement is used as an index and this value is 0.020 mmol/g-cat or less. Although a detailed mechanism thereof is not necessarily clear, the present inventors have presumed the mechanism as follows: a reaction substrate and product this time contain amino groups in their structures, and these amino groups are considered to react strongly with active sites on surface of catalyst. Specifically, since deammoniation reaction occurs, the generation of the impurities (by-products) is considered to occur. Thus, the amount at a desorption peak obtained by the present measurement is used as an index, and the value is sufficiently decreased so that the active sites on surface of catalyst which react strongly with NH₃ disappear. As a result, deammoniation reaction is suppressed, and selectivity is improved.

However, the description above merely states the contents of a possible cause of the catalyst of the present embodiment to exert excellent BAC selectivity. The mechanism of action of the present embodiment is not limited thereto.

The amount at a desorption peak is preferably 0.015 mmol/g-cat or less from the viewpoint mentioned above as well as the viewpoint of also more improving a BAC yield.

More specifically, the amount at a desorption peak can be measured on the basis of a method described in Examples mentioned later.

The amount at a desorption peak can be adjusted to the above range, for example, by producing the catalyst on the basis of a production method mentioned later.

The catalyst of the present embodiment preferably comprises a catalytic component and a support that supports the catalytic component from the viewpoint of catalyst strength.

### (Catalytic component)

The catalytic component according to the present embodiment is not particularly limited, and various known catalytic components can be adopted. Examples of the catalytic component according to the present embodiment include Ru, Rh, and Ni. One of these components may be used singly, or two or more thereof may be used in combination. In the present embodiment, the catalytic component preferably comprises Ru from the viewpoint of hydrogenation ability. Particularly preferably, the catalyst of the present embodiment comprises a catalytic component and a support that supports the catalytic component, and the catalytic component comprises Ru.

When the catalyst of the present embodiment comprises Ru, the content of Ru is preferably 0.1 to 10.0% by mass based on 100% by mass of the catalyst from the viewpoint of hydrogenation ability.

The content of Ru can be measured by X-ray fluorescence analysis (XRF). The content may be identified as a starting material charging ratio at the time of catalyst production.

### (Support)

The support according to the present embodiment is not particularly limited as long as the catalytic component can be supported thereon. Various known supports can be adopted. Examples of the support according to the present embodiment include alumina, diatomaceous earth, and carbon. One of these supports may be used singly, or two or more thereof may be used in combination. In the present embodiment, the support preferably comprises alumina from the viewpoint of catalyst strength.

### (Alkali metal)

The catalyst of the present embodiment preferably comprises an alkali metal from the viewpoint of hydrogenation ability. The alkali metal according to the present embodiment is discriminated from the catalytic component mentioned above and is not particularly limited by its type. Examples thereof include Li, Na, K, Rb, and Cs. One of these alkali metals may be used singly, or two or more thereof may be used in combination. The alkali metal according to the present embodiment preferably comprises at least one selected from the group consisting of Na, K, and Li from the viewpoint of hydrogenation ability. The alkali metal according to the present embodiment more preferably comprises at least one selected from the group consisting of Na and K from the viewpoint mentioned above as well as the viewpoint of also more improving a BAC yield.

When the catalyst of the present embodiment comprises the alkali metal, the content of the alkali metal is preferably 0.1 to 10.0% by mass based on 100% by mass of the catalyst from the viewpoint of hydrogenation ability. Particularly, when the catalyst of the present embodiment comprises Na, the content of Na is preferably 0.1 to 10.0% by mass based on 100% by mass of the catalyst from the viewpoint of hydrogenation ability. Particularly preferably, for the catalyst of the present embodiment, the Ru content in the catalyst is 0.1 to 10.0% by mass based on 100% by mass of the catalyst, and the Na content in the catalyst is 0.1 to 10.0% by mass based on 100% by mass of the catalyst.

The content of Na and the contents of other alkali metals can be measured on the basis of a method described in Examples mentioned later. The content of Na and the contents of other alkali metals may be identified as a starting material charging ratio at the time of catalyst production.

### <Method for producing catalyst>

A method for producing the catalyst of the present embodiment is not particularly limited as long as the catalyst of the present embodiment can be obtained. The catalyst of the present embodiment is preferably obtained by the following method: the preferred method for producing the catalyst of the present embodiment (hereinafter, also referred to as the "production method (A)") is a method for producing the catalyst of the present embodiment, comprising: a step (a) of reacting a catalytic component with a support to obtain a first reaction product; and a step (b) of adding an alkali metal to the first reaction product to obtain a second reaction product. The production method (A) thus configured can efficiently produce a catalyst that exerts excellent BAC selectivity.

### (Step (a))

In the step (a), a catalytic component is reacted with a support to obtain a first reaction product. Those listed in the section <Catalyst> can be appropriately adopted to the catalytic component and the support. The reaction of the catalytic component with the support can be carried out in accordance with, for example, but not limited to, an incipient wetness (IW) method, by adopting various known conditions.

In the present embodiment, the step (a) preferably comprises adsorbing a Ru starting material solution onto the support, then drying the support, and bringing the support into contact with a basic aqueous solution, from the viewpoint of the hydrogenation ability of the resulting catalyst. The Ru starting material solution preferably contains, for example, but not limited to, ruthenium chloride n-hydrate. The basic aqueous solution preferably contains, for example, but not limited to, sodium hydroxide.

### (Step (b))

In the step (b), an alkali metal is added to the first reaction product to obtain a second reaction product. The second reaction product may be used in itself as the catalyst of the present embodiment, or may be used as the catalyst of the present embodiment after performance such as hydrogenation ability is further adjusted through a step (c) mentioned later. Those listed in the section <Catalyst> can be appropriately adopted to the alkali metal. The addition of the alkali metal can be carried out in accordance with, for example, but not limited to, an incipient wetness (IW) method, by adopting various known conditions. The alkali metal may be added in the forms of, for example, but not limited to, carbonate, bicarbonate, nitrate, and hydroxide. The alkali metal according to the present embodiment preferably comprises at least one selected from the group consisting of Na, K, and Li from the viewpoint of the hydrogenation ability of the resulting catalyst.

### (Step (c))

The production method (A) preferably further comprises a step (c) of reducing the second reaction product to obtain a third reaction product. In the case of using the third reaction product as the catalyst of the present embodiment, hydrogenation ability tends to be higher. In the production method (A), the amount at a desorption peak measured by the ammonia temperature-programmed desorption method for the third reaction product is preferably 0.020 mmol/g-cat or less, more preferably 0.015 mmol/g-cat or less, from the same viewpoint as above.

The temperature condition of the step (c) is not particularly limited, and the step (c) is performed at a temperature of 160 to 360°C from the viewpoint of the hydrogenation ability of the resulting catalyst.

### <Method for producing bis(aminomethyl)cyclohexane>

The method for producing bis(aminomethyl)cyclohexane according to the present embodiment (hereinafter, also referred to as the "production method of the present embodiment") comprises a hydrogenation step of continuously bringing xylylenediamine and hydrogen into contact with each other in the presence of a catalyst having hydrogenation ability to obtain bis(aminomethyl)cyclohexane, wherein an amount at a desorption peak measured for the catalyst by the following ammonia temperature-programmed desorption method is 0.020 mmol/g-cat or less:

### (Ammonia temperature-programmed desorption method)

He gas is brought into contact with 150 mg of the catalyst at 500°C at a gas flow rate of 20 sccm for 30 minutes; subsequently, a mixed gas of 5% by volume of NH₃ and balance He is brought into contact with the catalyst at 50°C at a gas flow rate of 20 sccm for 10 minutes; and then, while He gas is brought into contact with the catalyst at a gas flow rate of 20 sccm, the temperature is increased at 10°C/min, followed by the detection of the desorption peak with a thermal conductivity detector.

The production method of the present embodiment thus configured can produce BAC with high BAC selectivity.

### (Hydrogenation step)

In the hydrogenation step according to the present embodiment, xylylenediamine and hydrogen are continuously brought into contact with each other in the presence of a catalyst having hydrogenation ability to obtain bis(aminomethyl)cyclohexane. Specifically, continuous hydrogenation of xylylenediamine is performed. Bis(aminomethyl)cyclohexane obtained by the production method of the present embodiment can be 1,3-bis(aminomethyl)cyclohexane, can be 1,4-bis(aminomethyl)cyclohexane, and can be a mixture thereof.

In the production method of the present embodiment, xylylenediamine, one of the starting materials, has three isomers, i.e., ortho, meta, and para forms, any of which can be used singly or as a mixture as the starting material. In the production method of the present embodiment, meta and para forms can be preferably adopted. Specifically, xylylenediamine preferably comprises metaxylylenediamine (hereinafter, also referred to as "MXDA") and/or paraxylylenediamine (hereinafter, also referred to as "PXDA").

In the production method of the present embodiment, the solvent that may be used is not particularly limited, and various known solvents can be adopted. Examples of the solvent according to the present embodiment include, but are not limited to, alkylamines and alkylenediamines. One of these solvents may be used singly, or two or more thereof may be used in combination. When any of the alkylamines and the alkylenediamines are used as the solvent, a substance that is liquid at ordinary temperature and has 1 to 18 carbon atoms is preferably selected because the solvent is separated by distillation from a reaction product and cyclically used, and therefore, is easily circulated in an industrial continuous production method. In this case, the generation of by-products is more suppressed, and the yield of the compound of interest tends to be more increased.

Examples of the alkylamines according to the present embodiment include, but are not limited to, methylamine, dimethylamine, trimethylamine, ethylamine, diethylamine, triethylamine, propylamine, dipropylamine, isopropylamine, diisopropylamine, butylamine, dibutylamine, tributylamine, hexylamine, cyclohexylamine, and 2-ethylhexylamine. One of these alkylamines may be used singly, or two or more thereof may be used in combination.

Examples of the alkylenediamines according to the present embodiment include, but are not limited to, ethylenediamine, propylenediamine, 1,4-butylenediamine, hexamethylenediamine, and bis(aminomethyl)cyclohexane. One of these alkylenediamines may be used singly, or two or more thereof may be used in combination.

In the present embodiment, bis(aminomethyl)cyclohexane obtained by catalytic reduction can be cyclically used. Therefore, the solvent according to the present embodiment preferably comprises 1,3-bis(aminomethyl)cyclohexane and/or 1,4-bis(aminomethyl)cyclohexane.

In the production method of the present embodiment, the mixed weight ratio of the alkylamines and the alkylenediamines to the starting material xylylenediamine (amount of xylylenediamine:total amount of alkylamines and alkylenediamines) is not particularly limited and is preferably 1:30 to 1:1.

Each of the alkylamines or the alkylenediamines may be used alone, or a mixture of the amines or the alkylenediamines may be used, or a mixture with an additional organic solvent may be used. Examples of the organic solvent that may be mixed include, but are not limited to, alcohols such as methanol, ethanol, isopropyl alcohol, and n-propyl alcohol.

In the hydrogenation step according to the present embodiment, the feeding condition of hydrogen is not particularly limited. The hydrogen pressure is preferably 5 MPa or higher and 15 MPa or lower, and the amount of hydrogen fed per mL of the catalyst is preferably 0.5 NmL/min or more and 2.5 NmL/min or less. The hydrogen pressure can be identified as a gauge pressure.

In the catalyst in the production method of the present embodiment, the amount at a desorption peak measured by the ammonia temperature-programmed desorption method is 0.020 mmol/g-cat or less, preferably 0.015 mmol/g-cat or less, from the viewpoint of hydrogenation ability. Those described in the section

### <Catalyst> can be appropriately adopted to the catalyst.

The catalyst in the production method of the present embodiment can comprise a catalytic component and a support that supports the catalytic component. Those listed in the section <Catalyst> can be appropriately adopted to the catalytic component and the support.

Preferably, the catalyst in the production method of the present embodiment comprises a catalytic component and a support that supports the catalytic component, and the catalytic component comprises Ru, from the viewpoint of hydrogenation ability.

In the catalyst in the production method of the present embodiment, the support preferably comprises alumina.

The catalyst in the production method of the present embodiment preferably comprises an alkali metal, in addition to the catalytic component and the support. Those listed in the section <Catalyst> can be appropriately adopted to the alkali metal. The alkali metal preferably comprises at least one selected from the group consisting of Na, K, and Li from the viewpoint of hydrogenation ability.

For the catalyst in the production method of the present embodiment, preferably, the Ru content in the catalyst is 0.1 to 10.0% by mass based on 100% by mass of the catalyst, and the alkali metal content in the catalyst is 0.1 to 10.0% by mass based on 100% by mass of the catalyst.

For the catalyst in the production method of the present embodiment, preferably, the Ru content in the catalyst is 0.1 to 10.0% by mass based on 100% by mass of the catalyst, and the Na content in the catalyst is 0.1 to 10.0% by mass based on 100% by mass of the catalyst.

Preferably, the catalyst in the production method of the present embodiment is obtained by a production method (A), and the production method (A) comprises: a step (a) of reacting the catalytic component with the support to obtain a first reaction product; and a step (b) of adding an alkali metal to the first reaction product to obtain a second reaction product. The second reaction product may be used in itself as the catalyst in the production method of the present embodiment, or may be used as the catalyst in the production method of the present embodiment after performance such as hydrogenation ability is further adjusted through a step (c) mentioned later.

In this context, those described in the section <Method for producing catalyst> can be appropriately adopted to the production method (A).

Specifically, the production method (A) further comprises a step (c) of reducing the second reaction product to obtain a third reaction product, and the amount at a desorption peak measured by the ammonia temperature-programmed desorption method for the third reaction product is preferably 0.020 mmol/g-cat or less, more preferably 0.015 mmol/g-cat or less, from the viewpoint of the hydrogenation ability of the resulting catalyst.

The step (a) preferably comprises adsorbing a Ru starting material solution onto the support, then drying the support, and bringing the support into contact with a basic aqueous solution, from the viewpoint of the hydrogenation ability of the resulting catalyst.

The alkali metal in the step (b) preferably comprises at least one selected from the group consisting of Na, K, and Li from the viewpoint of the hydrogenation ability of the resulting catalyst.

The step (c) is preferably performed at a temperature of 160 to 360°C from the viewpoint of the hydrogenation ability of the resulting catalyst.

The amount of the catalyst in the production method of the present embodiment is not particularly limited, and WHSV based on the amount of the starting material xylylenediamine fed can be 0.1 to 5.0 and is preferably 0.1 to 2.0. The amount described above can be preferably adopted in the case of using a catalyst carrying approximately 2% by mass of ruthenium as the catalyst of the present embodiment. Such a condition can also be adopted for other forms of the catalyst of the present embodiment.

In the hydrogenation step according to the present embodiment, the reaction temperature is not particularly limited. The reaction temperature is 50 to 150°C, preferably 80 to 130°C.

A reactor for carrying out the hydrogenation step in the present embodiment is not particularly limited. For example, a fixed-bed reactor can be used. In the case of performing continuous reaction using a solvent, the reactor according to the present embodiment may be configured such that, after a solution gas is separated from a reaction product with a gas-liquid separator, BAC obtained by catalytic hydrogenation can be separated and cyclically used, or may be configured such that a reaction product after gas-liquid separation can be subjected to the separation of the solvent by distillation in a solvent collection facility and cyclically used. In the present embodiment, BAC obtained by catalytic hydrogenation may be used as the solvent. In this case, a solution gas is preferably separated from the reaction product with a gas-liquid separator and then cyclically used directly.

### (Arbitrary step)

The production method of the present embodiment may comprise an arbitrary step, in addition to the hydrogenation step. The production method of the present embodiment may further comprise, for example, but not limited to, a purification step. The purification step involves, for example, distilling off alkylamines, alkylenediamines, and an organic solvent at ordinary pressure, followed by vacuum distillation. The compound of interest (BAC) can thereby be preferably separated from the reaction product.

### Examples

Hereinafter, the present embodiment will be described in more detail with reference to Examples. However, the scope of the present embodiment is not limited by these Examples.

### [Example 1]

### (Production of catalyst)

A catalytic component was supported onto alumina by the incipient wetness (IW) method using 20 g of alumina, 1 g of ruthenium chloride n-hydrate, and 8 g of water, followed by drying at 120°C to obtain a supported material 1. The supported material 1 was subjected to base treatment using 60 g of 1 M sodium hydroxide and 300 g of water (i.e., the supported material 1 was brought into contact with sodium hydroxide and water), followed by drying at 110°C to obtain a supported material 2. To this supported material, 1.0 g of NaHCO₃ and 8 g of water were added by the IW method, followed by drying at 110°C to obtain a catalyst of Example 1.

### (Amount of alkali metal in catalyst)

As a result of conducting X-ray fluorescence (XRF) analysis, the amount of the alkali metal (the amount of Na) contained in the catalyst was 1.7% by mass based on 100% by mass of the catalyst. Specifically, the amount of the alkali metal (the amount of Na) was 0.8 mmol/g-cat.

### (TPD measurement)

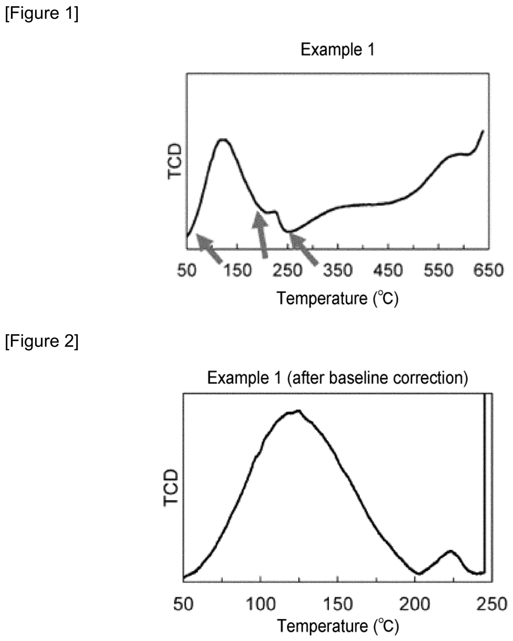

He gas was brought into contact with 150 mg of the catalyst at 500°C at a gas flow rate of 20 sccm for 30 minutes. Subsequently, a mixed gas of 5% by volume of NH₃ and balance He was brought into contact with the catalyst at 50°C at a gas flow rate of 20 sccm for 10 minutes. Then, while He gas was brought into contact with the catalyst at a gas flow rate of 20 sccm, the temperature was increased at 10°C/min, followed by the detection of the desorption peak with a thermal conductivity detector ("BEL-CAT B" from MicrotracBEL Corp.). Specifically, the desorption peak was calculated from a peak area on the higher temperature side obtained by vertically partitioning a peak that appeared at 50 to 350°C after multipoint baseline correction, and the multipoint baseline correction was carried out by a known method using peak start and end points. The amount at the desorption peak (mmol/g-cat) was determined by calibration pulse measurement using NH₃. Specifically, a value calculated using the value of a calibration factor (assuming that the gas species of the desorption peak was NH₃) was exploited on the basis of the method described in the manual of "BEL-CAT B". TPD measurement results of the catalyst of Example 1 are shown in Figure 1 (before multipoint baseline correction) and Figure 2 (after multipoint baseline correction). The arrows in each drawing correspond to positions (the peak start and end points were selected) corrected in the multipoint baseline correction. As a result, the amount at the desorption peak of Example 1 was 0.005 (mmol/g-cat).

### (Production of BAC)

The following hydrogenation reaction was carried out using an external heating-type fixed-bed flow reactor of 17 mmϕ in inside diameter × 320 mmL. First, hydrogen gas was brought into contact with 16 mL of the catalyst at 260°C at a gas flow rate of 100 mL/min for 7 hours to reduce the catalyst. Subsequently, a mixture consisting of a starting material metaxylylenediamine (4% by mass) and a solvent 1,3-bis(aminomethyl)cyclohexane (96% by mass) was fed at 45 g/hr into the reactor in the presence of this catalyst thus reduced, under conditions involving a catalytic amount of 16 mL, a reaction temperature of 100°C, a hydrogen pressure of 8.7 MPa (gauge pressure), and an amount of hydrogen fed of 25 NmL/min (1.6 NmL/min per mL of the catalyst).

### (Reaction performance)

After a lapse of 24 hours from the start of reaction, the reaction product was sampled for 1 hour and analyzed by gas chromatography. As a result, the BAC selectivity was 94.2%. The MXDA conversion rate was 88.9%. The gas chromatography analysis conditions were as described below, and quantification was performed by the area percentage method.
GC apparatus: Shimadzu GC2030
Column: HP-1MS (column of 30 m in length × 0.25 mm in inside diameter × 0.25 µm in film thickness)
Sample introduction temperature: 300°C
Split ratio: 10
Temperature program: The temperature is kept at 150°C for 5 minutes, then increased to 300°C (for 10 min), and then kept at 300°C for 10 minutes
Detector and detection temperature: Hydrogen flame ionization detector (FID), 300°C
Carrier gas: He (46.2 mL/min)
Injection volume: 0.2 µL

### (Reduction of catalyst and TPD measurement)

Aside from the above operation, hydrogen gas was brought into contact with catalyst 150 mg of the catalyst obtained in the section "Production of catalyst" (i.e., the catalyst before being used in the section "Production of BAC") at 260°C at a gas flow rate of 50 sccm for 30 minutes to reduce the catalyst. Subsequently, He gas was brought into contact with the catalyst thus reduced at 500°C at a gas flow rate of 20 sccm for 30 minutes. Subsequently, a mixed gas of 5% by volume of NH₃ and balance He was brought into contact with the catalyst at 50°C at a gas flow rate of 20 sccm for 10 minutes. Then, while He gas was brought into contact with the catalyst at a gas flow rate of 20 sccm, the temperature was increased at 10°C/min, followed by the detection of the desorption peak with a thermal conductivity detector ("BEL-CAT B" from MicrotracBEL Corp.). The multipoint baseline correction and the calculation of the amount at the desorption peak (mmol/g-cat) were carried out in the same manner as mentioned above. As a result, the amount at the desorption peak of the reduced catalyst was 0.005 (mmol/g-cat), which was well consistent with the value before reduction.

### [Example 2]

A catalyst of Example 2 was obtained in the same manner as in Example 1 except that 1.0 g of K₂CO₃ was used instead of 1.0 g of NaHCO₃ in the production of the catalyst of Example 1. The amount of the alkali metal was measured as to the obtained catalyst in the same manner as in Example 1, and TPD measurement was also carried out. As a result, the amount at the desorption peak of Example 2 was 0.002 (mmol/g-cat). BAC was further produced in the same manner as in Example 1 using the catalyst of Example 2, and reaction performance was evaluated. The BAC selectivity was 94.2%, and the MXDA conversion rate was 90.7%.

The results described above are shown in Table 1.

### [Example 3]

A catalyst of Example 3 was obtained in the same manner as in Example 1 except that 1.0 g of LiOH was used instead of 1.0 g of NaHCO₃ in the production of the catalyst of Example 1. The amount of the alkali metal was measured as to the obtained catalyst in the same manner as in Example 1, and TPD measurement was also carried out. As a result, the amount at the desorption peak of Example 3 was 0.019 (mmol/g-cat). BAC was further produced in the same manner as in Example 1 using the catalyst of Example 3, and reaction performance was evaluated. The BAC selectivity was 94.0%.

The results described above are shown in Table 1.

### [Example 4]

A catalyst of Example 4 was obtained in the same manner as in Example 1 except that 4.0 g of LiNO₃ was used instead of 1.0 g of NaHCO₃ in the production of the catalyst of Example 1. The amount of the alkali metal was measured as to the obtained catalyst in the same manner as in Example 1, and TPD measurement was also carried out. As a result, the amount at the desorption peak of Example 4 was 0.001 (mmol/g-cat). BAC was further produced in the same manner as in Example 1 using the catalyst of Example 4, and reaction performance was evaluated. The BAC selectivity was 94.3%. The BAC yield was higher than that of Example 3.

The results described above are shown in Table 1.

As a result of further carrying out the reduction of the catalyst and TPD measurement in the same manner as in Example 1 as to the catalyst of Example 4, the amount at the desorption peak of the catalyst thus reduced was 0.001 (mmol/g-cat), which was well consistent with the value before reduction.

### [Comparative Example 1]

The obtained supported material 2 was used directly as a catalyst of Comparative Example 1 without the addition of 1.0 g of NaHCO₃ in the production of the catalyst of Example 1.

The amount of the alkali metal was measured as to the catalyst of Comparative Example 1 in the same manner as in Example 1, and TPD measurement was also carried out. As a result, the amount at the desorption peak of Comparative Example 1 was 0.036 (mmol/g-cat). BAC was further produced in the same manner as in Example 1 using the catalyst of Comparative Example 1, and reaction performance was evaluated. The BAC selectivity was 91.8%. The BAC yield was lower than that of Example 1.

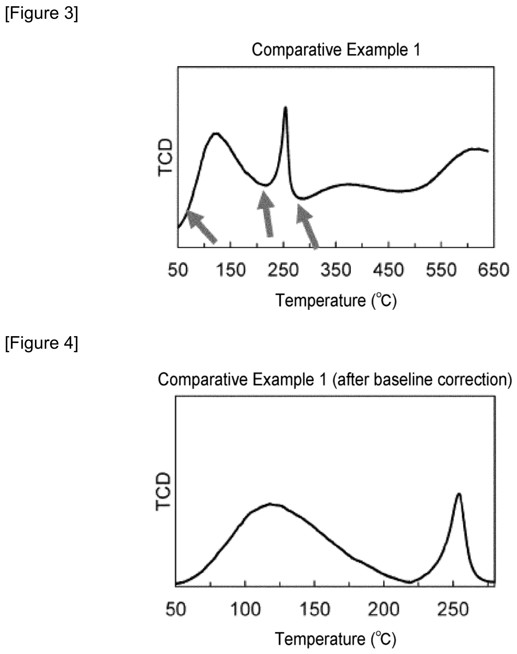

The results described above are shown in Table 1. TPD measurement results of the catalyst of Comparative Example 1 are shown in Figure 3 (before multipoint baseline correction) and Figure 4 (after multipoint baseline correction). The arrows in each drawing correspond to positions (the peak start and end points were selected) corrected in the multipoint baseline correction.

### [Comparative Example 2]

A catalyst was produced under the same production conditions as in Example 10 of Patent Literature 1 and used as a catalyst of Comparative Example 2. Specifically, a catalytic component was supported onto alumina by the equilibrium adsorption method (dipping for 12 hours) using 10 g of alumina, 1 g of ruthenium chloride n-hydrate, and 200 g of water, followed by drying at 100°C for 12 hours to obtain a supported material a. The supported material a was fired at 500°C in air to obtain a supported material b. Nitrogen gas containing 10% hydrogen was brought into contact with the supported material b at 150°C for 10 hours to obtain a supported material c. Na was added to the supported material c by the equilibrium adsorption method using 0.5 g of Na₂CO₃ and 200 g of water, followed by drying to obtain a catalyst of Comparative Example 2.

TPD measurement was carried out as to the catalyst of Comparative Example 2 in the same manner as in Example 1.

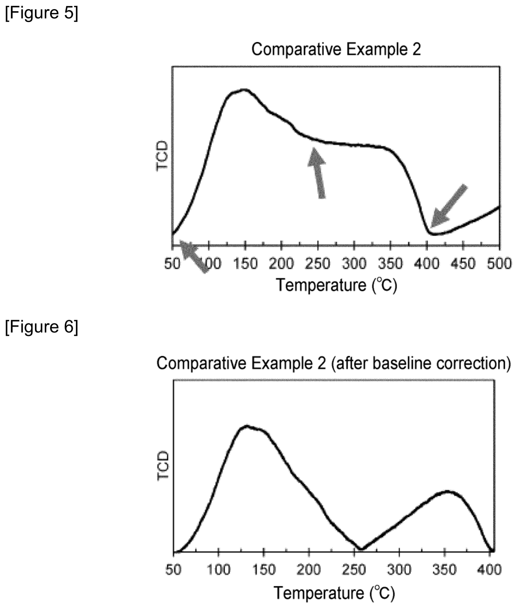

TPD measurement results of the catalyst of Comparative Example 2 are shown in Figure 5 (before multipoint baseline correction) and Figure 6 (after multipoint baseline correction). The arrows in each drawing correspond to positions (the peak start and end points were selected) corrected in the multipoint baseline correction. As a result, the amount at the desorption peak of Comparative Example 2 was 0.054 (mmol/g-cat).

### [Comparative Example 3]

A catalyst was produced under the same production conditions as in Comparative Example 1 of Patent Literature 1 and used as a catalyst of Comparative Example 3.

TPD measurement was carried out as to the catalyst of Comparative Example 3 in the same manner as in Example 1.

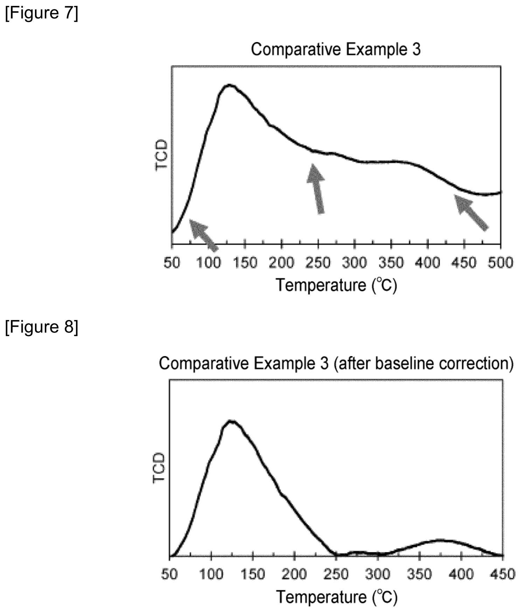

TPD measurement results of the catalyst of Comparative Example 3 are shown in Figure 7 (before multipoint baseline correction) and Figure 8 (after multipoint baseline correction). The arrows in each drawing correspond to positions (the peak start and end points were selected) corrected in the multipoint baseline correction. As a result, the amount at the desorption peak of Comparative Example 3 was 0.023 (mmol/g-cat).

**[Table 1]**

| | | Example 1 | Example 2 | Example 3 | Example 4 | Comparative Example 1 |
|---|---|---|---|---|---|---|
| Alkali metal addition | Amount of NaHCO₃ added (g) | 1.0 | - | - | - | - |
| | Amount of K₂CO₃ added (g) | - | 1.0 | - | - | - |
| | Amount of LiOH added (g) | - | - | 1.0 | - | - |
| | Amount of LiNO₃ added (g) | - | - | - | 4.0 | - |
| Evaluation results | Amount of alkali metal in catalyst (wt%) | 1.7 | 2.0 | 1.2 | 3.2 | 0.7 |
| | Amount of alkali metal in catalyst (mmol/g-cat) | 0.8 | 0.6 | 1.3 | 3.5 | 0.3 |
| | Amount at desorption peak in NH₃-TPD (mmol/g-cat) | 0.005 | 0.002 | 0.019 | 0.001 | 0.036 |
| | BAC selectivity (%) | 94.2 | 94.2 | 94.0 | 94.3 | 91.8 |

The methods for producing the catalysts according to Comparative Examples 2 and 3 were evaluated as having the difficulty in controlling the amount of the alkali metal incorporated into the catalysts because the alkali metal was added by the equilibrium adsorption method; and as a result, the amount at the desorption peak tended to be also difficult to control. By contrast, the methods for producing the catalysts according to Examples 1 to 4 were evaluated as being able to easily control the amount of the alkali metal incorporated into the catalysts as compared with Comparative Examples 2 and 3 because the alkali metal was added by the IW method after base treatment; as a result, the amount at the desorption peak tended to be also easy to control.

The BAC selectivity was confirmed to be higher in all of Examples 1 to 4 than in Comparative Example 1. The BAC yields of Examples 1 and 2 were higher than those of Examples 3 and 4, and tendency for more improving not only the selectivity but the yield was found in Na and K used as the alkali metal. In such comparison, when a difference in BAC selectivity or BAC yield was more than 0.1, the difference was evaluated as being significant in light of recent levels of performance required for catalysts.

### [Comparative Example 4]

BAC was produced in the same manner as in Comparative Example 1 except that metaxylylenediamine (4% by mass) was changed to paraxylylenediamine (4% by mass) as a starting material and the solvent was changed from 1,3-bis(aminomethyl)cyclohexane to 1,4-bis(aminomethyl)cyclohexane, in the BAC production of Comparative Example 1. Reaction performance was evaluated (BAC selectivity and PXDA conversion rate were calculated).

### [Example 5]

BAC was produced in the same manner as in Comparative Example 4 except that the catalyst of Example 1 was used instead of the catalyst of Comparative Example 4 in the BAC production of Comparative Example 4. Reaction performance was evaluated. The BAC selectivity of Example 5 was higher by 0.5% than that of Comparative Example 4. The PXDA conversion rate of Example 5 was higher by 4.0% than that of Comparative Example 4. The yield of Example 5 was higher by 4.2% than that of Comparative Example 4.

### [Example 6]

BAC was produced in the same manner as in Comparative Example 4 except that the catalyst of Example 2 was used instead of the catalyst of Comparative Example 4 in the BAC production of Comparative Example 4. Reaction performance was evaluated. The BAC selectivity of Example 6 was higher by 1.1% than that of Comparative Example 4. The PXDA conversion rate of Example 6 was higher by 4.2% than that of Comparative Example 4. The yield of Example 6 was higher by 5.0% than that of Comparative Example 4.

As is evident from the comparison of Examples 5 and 6 with Comparative Example 4, the catalysts according to Examples 1 and 2 exerted higher catalyst performance than that of the catalyst according to Comparative Example 1 even when PXDA was used as a starting material in BAC production.

The present application claims priority to the Japanese patent application filed on March 31, 2023 (Japanese Patent Application No. 2023-058039), the contents of which are incorporated herein by reference.

## Claims

1. A catalyst for producing bis(aminomethyl)cyclohexane, the catalyst having hydrogenation ability, wherein
an amount at a desorption peak measured for the catalyst by the following ammonia temperature-programmed desorption method is 0.020 mmol/g-cat or less:
(Ammonia temperature-programmed desorption method)
He gas is brought into contact with 150 mg of the catalyst at 500°C at a gas flow rate of 20 sccm for 30 minutes; subsequently, a mixed gas of 5% by volume of NH₃ and balance He is brought into contact with the catalyst at 50°C at a gas flow rate of 20 sccm for 10 minutes; and then, while He gas is brought into contact with the catalyst at a gas flow rate of 20 sccm, the temperature is increased at 10°C/min, followed by the detection of the desorption peak with a thermal conductivity detector.

2. The catalyst according to claim 1, wherein
the catalyst comprises a catalytic component and a support that supports the catalytic component, and
the catalytic component comprises Ru.

3. The catalyst according to claim 2, wherein
a Ru content in the catalyst is 0.1 to 10.0% by mass based on 100% by mass of the catalyst, and
a Na content in the catalyst is 0.1 to 10.0% by mass based on 100% by mass of the catalyst.

4. The catalyst according to claim 3, wherein the support comprises alumina.

5. A method for producing the catalyst according to claim 1, comprising:
a step (a) of reacting a catalytic component with a support to obtain a first reaction product; and
a step (b) of adding an alkali metal to the first reaction product to obtain a second reaction product.

6. The method for producing the catalyst according to claim 5, further comprising
a step (c) of reducing the second reaction product to obtain a third reaction product, wherein
an amount at a desorption peak measured by the ammonia temperature-programmed desorption method for the third reaction product is 0.020 mmol/g-cat or less.

7. The method for producing the catalyst according to claim 6, wherein the step (c) is performed at a temperature of 160 to 360°C.

8. The method for producing the catalyst according to claim 5, wherein the step (a) comprises adsorbing a Ru starting material solution onto the support, then drying the support, and bringing the support into contact with a basic aqueous solution.

9. The method for producing the catalyst according to claim 5, wherein the alkali metal comprises at least one selected from the group consisting of Na, K, and Li.

10. A method for producing bis(aminomethyl)cyclohexane, comprising a hydrogenation step of continuously bringing xylylenediamine and hydrogen into contact with each other in the presence of a catalyst having hydrogenation ability to obtain bis(aminomethyl)cyclohexane, wherein
an amount at a desorption peak measured for the catalyst by the following ammonia temperature-programmed desorption method is 0.020 mmol/g-cat or less:
(Ammonia temperature-programmed desorption method)
He gas is brought into contact with 150 mg of the catalyst at 500°C at a gas flow rate of 20 sccm for 30 minutes; subsequently, a mixed gas of 5% by volume of NH₃ and balance He is brought into contact with the catalyst at 50°C at a gas flow rate of 20 sccm for 10 minutes; and then, while He gas is brought into contact with the catalyst at a gas flow rate of 20 sccm, the temperature is increased at 10°C/min, followed by the detection of the desorption peak with a thermal conductivity detector.

11. The method for producing bis(aminomethyl)cyclohexane according to claim 10, wherein
the catalyst comprises a catalytic component and a support that supports the catalytic component, and
the catalytic component comprises Ru.

12. The method for producing bis(aminomethyl)cyclohexane according to claim 11, wherein
the catalyst is obtained by a production method (A), and
the production method (A) comprises:
a step (a) of reacting the catalytic component with the support to obtain a first reaction product; and
a step (b) of adding an alkali metal to the first reaction product to obtain a second reaction product.

13. The method for producing bis(aminomethyl)cyclohexane according to claim 12, wherein
the production method (A) further comprises a step (c) of reducing the second reaction product to obtain a third reaction product, wherein
an amount at a desorption peak measured by the ammonia temperature-programmed desorption method for the third reaction product is 0.020 mmol/g-cat or less.

14. The method for producing bis(aminomethyl)cyclohexane according to claim 13, wherein the step (c) is performed at a temperature of 160 to 360°C.

15. The method for producing bis(aminomethyl)cyclohexane according to claim 14, wherein the step (a) comprises adsorbing a Ru starting material solution onto the support, then drying the support, and bringing the support into contact with a basic aqueous solution.

16. The method for producing bis(aminomethyl)cyclohexane according to claim 15, wherein the alkali metal comprises at least one selected from the group consisting of Na, K, and Li.

17. The method for producing bis(aminomethyl)cyclohexane according to claim 16, wherein
a Ru content in the catalyst is 0.1 to 10.0% by mass based on 100% by mass of the catalyst, and
a Na content in the catalyst is 0.1 to 10.0% by mass based on 100% by mass of the catalyst.

18. The method for producing bis(aminomethyl)cyclohexane according to claim 17, wherein the support comprises alumina.
